# EUROPEAN PATENT SPECIFICATION

(11) **EP 1 019 401 B1**
(45) Date of publication and mention of the grant of the patent: **16.01.2008**
(21) Application number: 98920543.0
(22) Date of filing: 20.04.1998
(51) Int. Cl.: C07D 471/04, C07D 471/14, C09K 11/00, C07D 487/14

(54) **COMPLEX COMPRISING A RARE-EARTH METAL ION AND A COMPLEXING MOIETY**
SELTENE ERDEN UND KOMPLEX GRUPPE ENTHALTENDE KOMPLEX
COMPLEXE COMPRENANT UN ION METALLIQUE DU GROUPE DES TERRES RARES ET UNE FRACTION COMPLEXANTE

(30) Priority: 25.04.1997 EP 97201236
(43) Date of publication of application: 19.07.2000
(73) Proprietor: Koninklijke Philips Electronics N.V., 5621 BA Eindhoven (NL)
(72) Inventor: VERHOEVEN, Jan, Willem, NL-1541 TW Koog aan de Zaan (NL); VAN DER TOL, Erik, Boudewijn, NL-1945 RK Beverwijk (NL); STEEMERS, Frank, Johannes, NL-7543 VC Enschede (NL); VERBOOM, Willem, NL-7671 WB Vriezenveen (NL); REINHOUDT, David, Nicolaas, NL-7552 GD Hengelo (NL); HOFSTRAAT, Johannes, Willem, NL-7006 VG Doetinchem (NL)
(74) Representative: Baele, Ingrid Albertina F.M.
(86) International application number: PCT/EP1998/002359
(87) International publication number: WO 1998/049163

(56) References cited:
- EP-A- 0 171 978
- CHEMICAL ABSTRACTS, vol. 109, no. 10, 5 September 1988 Columbus, Ohio, US; abstract no. 84977d, R.N. SHCHELOKOV ET AL.: "Rare earth nitrato complexes with 1,10-phenanthroline in the outer sphere" page 812; XP002041926 & ZH. NEORG. KHIM., vol. 33, no. 4, 1988, pages 867-74,
- CHEMICAL ABSTRACTS, vol. 111, no. 18, 1989 Columbus, Ohio, US; abstract no. 163397j, E.F. GUDGIN TEMPELTON: "Spectroscopic characterization of 1,10-phenanthroline-2,9-dicarboxylic acid and its complexes with europium(III): luminiscent europium chelates useful for analytical applications" page 656; XP002041927 & J. LUMIN., vol. 43, no. 4, 1989, pages 195-205,

## Description

The invention pertains to a complex comprising a rare-earth metal ion and a complexing moiety, a process for the preparation of the same, an optical device, and a diagnostic kit comprising said complex.

Molecular systems that combine specific binding ability and long-lived luminescence are of great interest for application as chemosensors and luminescence probes. Lanthanide ions in particular are of interest, because of their relatively long luminescence lifetime (up to ms) and their emission in the visible part of the spectrum. Such lanthanide based probes have been used for a wide range of applications. The disadvantage of the low extinction coefficient of lanthanide ions can be overcome by application of a sensitizer ("antenna") moiety. Recently, also near-IR luminescent lanthanide probes have been proposed, which make use of Nd³⁺ or Yb³⁺ as luminophores, both ions which emit in the region beyond 800 nm and are excitable in the visible part of the spectrum. With the same approach the Er³⁺ ion, which gives luminescence in the 1530 nm range, could be excited. Such complexes are also of interest for application in optical devices, such as optical amplifiers and lasers.

The challenge is to optimize this process in such a way that the sensitizer is able to sensitize multiple lanthanide ions (Eu³⁺, Tb³⁺, Sm³⁺, Dy³⁺, Nd³⁺, Yb³⁺, Er³⁺, etc.) with high luminescence efficiencies, allowing multi-probe analysis. Moreover, the sensitizer should have an absorption in the near-UV/VIS area to allow the use of inexpensive glass optics and simple excitation sources (e.g., Hg-lamp). In view of the relatively short wavelength of Tb³⁺ this allows only a small excitation window for effective sensitization. The sensitization of Tb³⁺ in particular is desirable, because of its high intrinsic quantum yield and its relatively low susceptibility to non-radiative quenching effects.

In European patent application EP 171,978 the compound 4,5,9,14-tetraaza-(1,2,3,4)-dibenzanthracene (TADDA) has been disclosed which forms a complex with the rare-earth metal ion europium(III). However, this complex has a low luminescence quantum yield.

In European patent application EP 493,745 a fluorescent compound has been disclosed which forms a complex with rare-earth metal ions. Such compounds are macro-cyclic compounds containing two phenanthrene moieties forming a cage structure in which the rare-earth metal ion can be complexed. These compounds were obtained by relatively complex synthetic methods, and it further appeared that they have a limited applicability and sometimes low quantum yields. There is, however, a need for simple compounds which strongly complex with rare-earth metal ions, have high quantum yields, and can be used for IR as well as for UV luminescence.

It has now been found that suitable complexes are complexes comprising a rare-earth metal ion and the complexing moiety of the general formula: 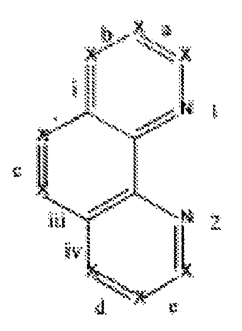 characterized in that
X is independently CH or N, and the bonds a, b, c, d, and e, and the combination of bonds i/ii and iii/iv are optionally condensed with a benzene group or a condensed aromatic moiety,
wherein aromatic carbon atoms may be replaced by nitrogen atoms and wherein the complexing moiety may be substituted with C₁₋₆ alkyl, C₂₋₆ alkenyl, C₂₋₆ alkynyl, C₃₋₄ alkylene,
CN, halogen, COOH, C₁₋₃ alkyl-COOH, NO₂, NH₂, or a pending group for further functionalization or complexation

Extensive studies aimed at direct coordination have made use of bipyridine-type complexes. Lehn et al., Angew. Chem., Int. Ed. Engl., 29 (1990), 1304, employed cryptates in which bipyridine ("the antenna") is incorporated into the macrocyclic structure. However, in aqueous solution the Eu³⁺ and Tb³⁺ cryptates convert only about 1% of the incident UV photons into emitted visible photons. Hemmilä et al., Helv. Chim. Acta., 76 (1993), 1361 investigated terpyridine structures and picolinic acid derivatives (J. Chem. Soc., Perkin Trans., 2 (1995), 995) in which the coordinating pyridine moiety serves as sensitizer, giving high quantum yields for Eu³⁺ and Tb³⁺.

It has now been found that in addition to the N atoms numbered 1 and 2, the addition of at least one other N atom in one of the aromatic nuclei, and preferably two other N atoms in the aromatic nuclei, considerably improves the quantum yield of the complex and the extinction coefficient of the sensitizer.

The presently claimed compounds comprise a rare-earth metal ion and the complexing moiety of the general formula: 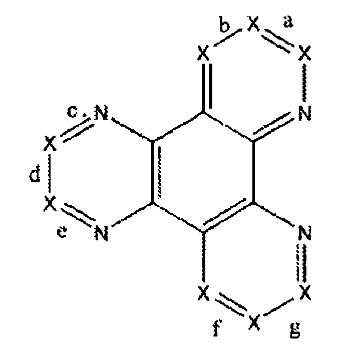 wherein X is independently CH or N, and the bonds a, b, c, d, e, f, and g are optionally condensed with a benzene group or a condensed aromatic moiety, wherein aromatic carbon atoms may be replaced by nitrogen atoms and wherein the complexing moiety may be substituted with C₁₋₆ alkyl, C₂₋₆ alkenyl, C₂₋₆ alkynyl, C₃₋₄ alkylene, CN, halogen, COOH, C₁₋₃ alkyl-COOH, NO₂, NH₂, or a pending group for further functionalization or complexation, the pending group comprising a coupling moiety being selected from the group consisting of isothiocyanate, activated ester, epoxide, acetic anhydride, maleimide, halogenoacetamide, amino, thiol, hydrazide, aldehyde, isocyanate, acid chloride, hydroxy, a group for forming a complex with a rare-earth metal ion where the group is a nitrogen-containin, a sulfur-containing or a phosphorous-containing group or calixarene.

The term C₁₋₆ alkyl means a branched or unbranched alkyl group with 1 to 6 carbon atoms, such as methyl, ethyl, propyl isopropyl, butyl, neopentyl, and hexyl. The term C₁₋₃ alkyl in alkyl-COOH means methyl, ethyl, or propyl. Methyl is the preferred alkyl group. The terms C₂₋₆ alkenyl and C₂₋₆ alkynyl mean, respectively, an alkenyl and alkynyl group with 2 to 6 carbon atoms. Examples are ethenyl, propenyl, ethynyl, propynyl, and the like. The C₃₋₄ alkylene group together with the aromatic group to which it is bonded forms a 5- or 6-membered ring.
The term halogen means fluorine, chlorine, bromine, or iodine.

The term pending group for further functionalization or complexation means any pending group which is used in the art to couple with peptides, proteins, carbohydrates, or nucleotides, or which are able to form a complex with rare-earth metal ions. Such pending groups possess an atom or atoms (coupling moiety) which can be used to couple with other groups or a group or groups which can be used to complex with rare-earth metal ions. Usual coupling moieties are, for instance, isothiocyanate, activated ester (e.g., N-hydroxysuccinimidylester), epoxide, acetic anhydride, and maleimide for coupling to amino groups, amino for coupling to isothiocyanate, activated ester, epoxide, acetic anhydride, and maleimide groups, maleimide and halogenoacetamide for coupling to thiol groups, thiol for coupling to maleimide and halogenoacetamide groups, hydrazide for coupling to aldehyde groups, aldehyde for coupling to hydrazide groups, isocyanate and acid chloride for coupling to hydroxy, amino, thiohydroxy, and carboxylic groups, and hydroxy for coupling to isocyante and acid chloride groups. Groups that can form complexes with rare-earth metal ions are carboxylic acids, and nitrogen, sulfur, and phosphorous containing groups. It is of particular advantage to use pending groups which comprise calixarene, or similar groups.

The condensed aromatic group is a group such as naphthalene, phenalene, naphthacene, perylene, pyrene, phenanthrene, anthracene, acenaphthylene, indene, benzanthracene, naphthoperylene, and the like. Preferably, the complexing moiety is condensed with a benzene or a naphthalene group.

The complexes can be applied in optical devices and diagnostic kits. Alternative applications for the described complexes are in optical amplification or as active material in a laser, or for security printing (bank notes etc.).

The invention is further illustrated by the following examples.

### Example 1

### Synthesis of Diazatriphenylene

Incorporation of N atoms in a cis-facile manner into the triphenylene structure gives a way to coordinate lanthanide atoms. To this end diazatriphenylene (3) was synthesized using a slightly modified literature procedure (Thang, D. C.; Jacquignon, P.; Dufour, M. J. Heterocyclic Chem., 13 (1976), 641) via a double Skraup reaction on 2,3-diaminonaphthalene(1). Twice performing a Michael addition on acroleine (obtained by dehydration of glycerol with sulfuric acid), followed by an acid catalyzed cyclization, gave diamine (2) in 51% yield. Diamine (2) was oxidized with arsenic pentoxide to give diazatriphenylene (3) in quantitative yield (Scheme 1). Functionalization of 3 via a nitration reaction to obtain nitrodiazatriphenylene (4) was performed using literature procedures (Richardson, F. S. Chem. Rev., 82 (1982), 541).

A detailed description of the synthesis follows:

To concentrated sulfuric acid (15 ml) was added dropwise dry glycerol (5 g). The mixure was stirred at room temperature for 5 min. Diamino-2,3-naphthalene (5 g) was added in portions. During this addition the mixture was kept in the liquid state by heating with a metal bath. After the addition was complete arsenic pentoxide (6.5 g) and sulfuric acid (30 ml) were added. The mixture was heated at 130-140°C for 2 h. The mixture was allowed to cool down to room temperature and poured out in water (100 ml) and crushed ice (100 g). The pH of the solution was adjusted to 9. The brown precipitate was filtered off, wereupon the residue was dissolved in ethyl acetate (100 ml). The organic solution was flushed over an aluminum oxide column (neutral, eluent: ethyl acetate). The product was obtained as a yellow solid: mp. 183-184°C (lit. 183°C).

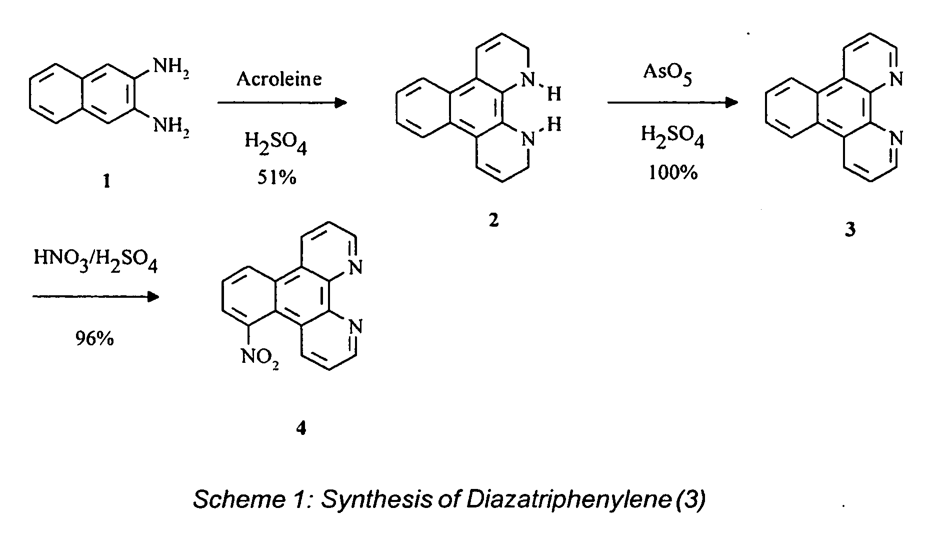

### Example 2

### Synthesis of tetraazatriphenylenes and derivatives from 1,10-phenanthroline-4,5-quinone

The tetraazatriphenylene derivatives (6-11) were synthesized starting from 1,10-phenanthroline-4,5-quinone (5), obtained from the oxidation of 1,10-phenanthroline monohydrate with bromine *(in situ* generated from sulfuric acid and sodium bromide) and nitric acid, according to literature procedures (Gillard, R. D.; Hill, R. E. E.; Maskill, R. J. Chem. Soc. (A) 1970, 1447). Subsequently the Schiff-base was formed with the diamines o-diaminobenzene, *cis*-diaminocyclohexane, diaminomalononitrile or ethylenediamine, and 1,10-phenanthroline-4,5-quinone in ethanol (or tetrahydrofuran), which after an oxidation step for compounds 6, 8, 10 and 11 (oxygen from the air), provided the tetraazatriphenylenes (6-11) in yields ranging from 80-84%. Subsequent heating of the diol with triethylamine as a base in N,N-dimethylformamide (DMF) gave the dicyanotetraazatriphenylene (7) in 80% yield.

The following products were prepared:

### Dipyrido[3,2-f:2', 3'-h]quinoxaline(6)

To a solution of 1,10-phenanthroline-4,5-quinone(0.60 g, 2.85 mmoles) in tetrahydrofuran (600 ml) was added dropwise ethylenediamine (0.19 g, 2.85 mmoles) dissolved in tetrahydrofuran (10 ml). Molecular sieves (3A) were added, and the solution was refluxed for 4 h. The solution was concentrated *in vacuo.* The slightly yellow solid was dissolved in dichloromethane (100 ml) and washed with 1 N hydrochloric acid (50 ml). The aqueous solution was adjusted to pH 9.0 and cooled on an ice bath. The precipitate was filtered, and after column chromatography (aluminum oxide, dichloromethane) and trituation with benzene compound 6 was obtained as a pure white solid: Yield 92%; mp > 300°C.

### 2,3-Dicyano-dipyrido[3,2-f:2',3'-h]quinoxaline(7)

To a solution of 1,10-phenanthroline-4,5-quinone(0.85 g, 4.0 mmoles) in tetrahydrofuran (100 ml) was added dicyanomaleonitrile (0.43 g, 4.0 mmoles). Molecular sieves (3A) were added, and the solution was refluxed for 1 h. The solution was filtered and concentrated *in vacuo.* The slightly red solid was trituated with dichloromethane. The solid was re-dissolved in DMF (50 ml), and 2 equivalents triethylamine were added under heating at 90°C for 30 min. The solvent was removed *in vacuo.* The solid was re-dissolved in dichloromethane (100 ml) and washed with 1 N hydrochloric acid (100 ml). Compound 7 was obtained after column chromatography (aluminum oxide, dichloromethane) and trituation with hexane/dichloromethaneas a pure white solid: Yield 71 %; mp > 300°C.

### Cis-2,3-cyclohexane-dipyrido[3,2-f:2',3'-h]quinoxaline(8)

To a solution of 1,10-phenanthroline-4,5-quinone (0.99 g, 4.7 mmoles) in ethanol was added *cis-*diaminocyclohexane (0.53 g, 4.6 mmoles). Molecular sieves (3A) were added, and the solution was refluxed for 1 h. The solution was concentrated *in vacuo,* and the obtained solid was subjected to column chromatography (aluminum oxide, dichloromethane). The solid was triturated with dichloromethane/hexane and crystallized from benzene to obtain compound 8 as a white pure solid: Yield 86%; mp 292-295°C.

### 2,3-(1,2-diaminobenzene)-dipyrido[3,2-f:2', 3'-h]quinoxaline (9)

Compound 9 was prepared and purified as described for compound 8, starting from 1,10-phenantholine-4,5-quinone(0.6 g, 2.9 mmoles) and 1,2-diaminobenzene (0.3 g, 2.9 mmoles). Yield 81 %.

### 3-[2-dipyrido[3,2-f:3',2'-h]quinoxaline]proponicacid (10)

To a solution of 1,10-phenanthroline-4,5-quinone(50 mg, 0.24 mmole) in ethanol (150 ml) was added DL-γ-ornithine (48 mg, 0.24 mmole). The solution was refluxed for 4 h and concentrated *in vacuo.* The solid was dissolved in ethanol/water (3/1) and purified by column chromatography (aluminum oxide, eluent ethanol/water : 3/1 + 1 % triethylamine). The slightly brown solid was triturated with ethyl acetate to obtain 10 as a pure white solid: Yield 53 %.

### 2-Methyl-dipyrido[3,2-f:2', 3'-h]quinoxaline(11)

Prepared in the same way as described for compound 8. Yield 83 %; mp. 271°C.

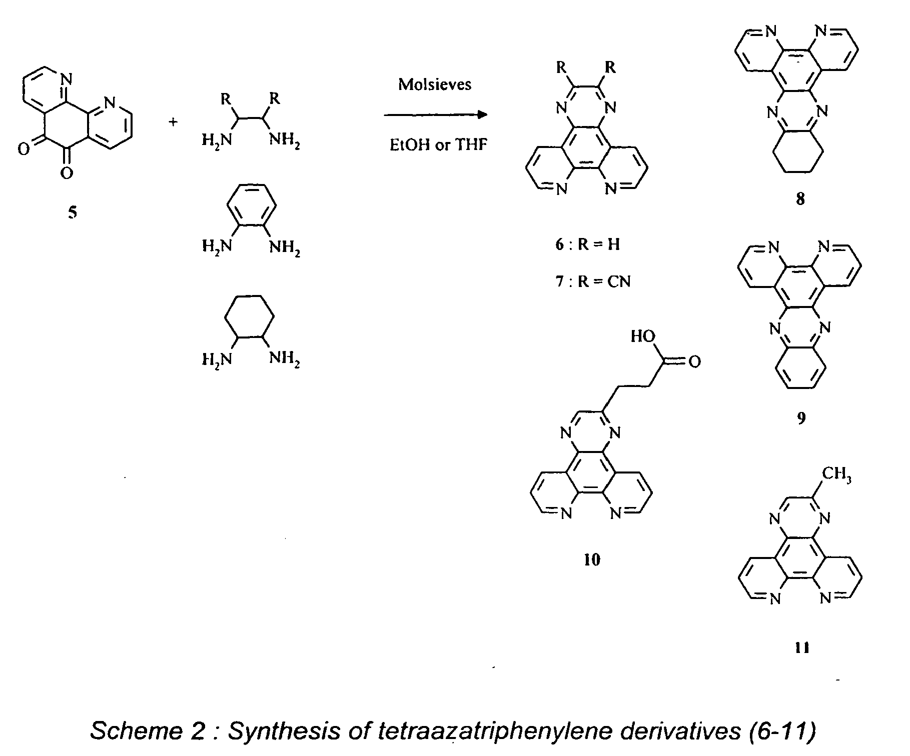

### Example 3

### Monofunctionalization of tetraazatriphenylene with 1,10-phenanthroline-4,5-quinone as starting material

Tetraazatriphenylene (16) was obtained by the reaction of diamine (15) and 1,10-phenanthroline-4,5-quinone 5 in a yield of 83%. For this purpose the diamine (15) was synthesized starting from DL-2,3-diaminopropionic acid (12) via a Boc-protection (13)-amidation (14)-Boc-deprotection (15) sequence as depicted in Scheme 3.

The detailed reaction is as follows:

### D, L-N, N'-di-tert-Boc-2, 3-diaminoproponicacid (13).

To a solution of D,L-2,3-diaminoproponicacid (12) (5.0 g) and triethylamine (18 g, 0.18 mmole) in 1,4-dioxane and water (75 ml, 1/1) was added di-*tert-*butyl dicarbonate (17.08 g, 78.3 mmoles). The solution was stirred at room temperature for 10 h. After completion of the reaction ethylacetate (100 ml) and 1 N hydrochloric acid (100 ml) were added. The organic layer was washed with brine (2 x 100 ml). The solvent was dried and removed *in vacuo*, whereupon product 13 was obtained as a white solid: Yield 91%; mp 162-164°C.

### D,L-N,N'-di-tert-Boc-2,3-diaminoproponicacid dodecyl amide (14)

To a solution of acid (13) (4.1 g, 13.3 mmoles) in tetrahydrofuran was added 1,1'-carbonyldiimidazole (3.0 g, 13.3 mmoles), 4-dimethylaminopyridine (catalyst, 0.1 g), and dodecylamine (2.7 g, 13.3 mmoles). The solution was stirred overnight and concentrated *in vacuo.* To the residue was added dichloromethane (100 ml), and the whole was washed with 1 N hydrochloric acid (aq.), followed by a standard work-up. The product was purified by column chromatography (silica gel, dichloromethane) to obtain pure 14 in 85% yield; mp 103-105°C.

### D,L-2,3-Diaminoproponicacid dodecyl amide (15).

To a solution of 10% sulfuric acid/dioxane (75 ml) was slowly added 14 (0.80 g, 1.7 mmoles) at 0°C. After the addition was complete, the mixture was stirred at room temperature for 4 h. The formed precipitate was filtered, dissolved in dichloromethane (100 ml), and washed subsequently with 1 M sodium hydroxide (aq) (50 ml) and brine (50 ml), followed by a standard work-up. The product was obtained, after trituration with MeOH, as a white pure solid: mp. 88-90 °C; Yield 77%.

### 2-Dipyrido[3,2-f:2',3'-h]quinoxalineacid dodecyl amide (16).

Prepared and purified similar to compound 8 starting from 1,10-phenantholine-4,5-quinone (0.50 g, 1.06 mmoles) and diamine 15 (0.50 g, 1.06 mmoles). Yield 81%; mp. 172-174°C.

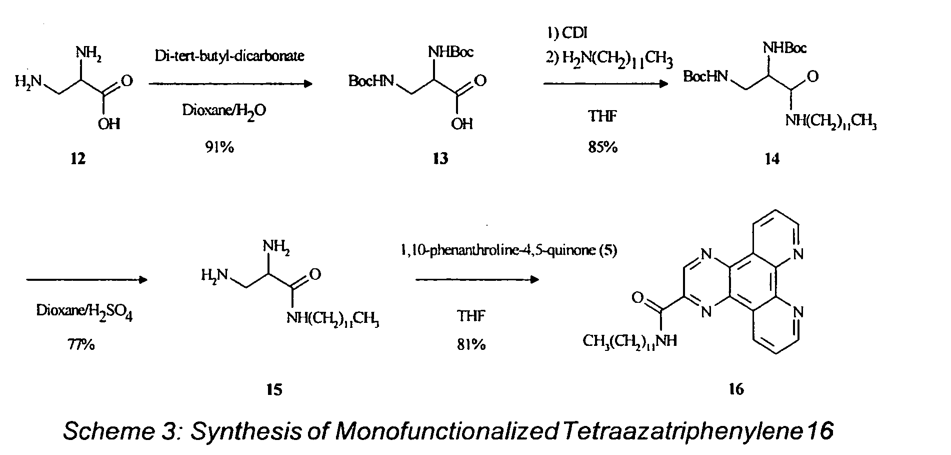

### Example 4

### Synthesis of tetraazatriphenylenesfrom 4,7-phenanthroline-4,5-quinone

### Cis-2,3-cyclohexane-dipyrido[2,3-f:3', 2'-h]quinoxaline (17)

Prepared according to a literature procedure starting from 1,2-*cis-*diaminocyclohexane (0.53 g, 4.6 mmole) and 4,7-phenanthroline-4,5-quinone(0.99 g, 4.7 mmoles).

### 3-[2-dipyrido[2,3-f:3',2'-h]quinoxaline]proponicacid (18).

Prepared similar to compound 16 starting from 4,7-phenanthroline-4,5-quinone (50 mg, 0.24 mmole) and DL-γ-ornithine (48 mg, 0.24 mmole). Yield 55%; mp. 230-231°C.

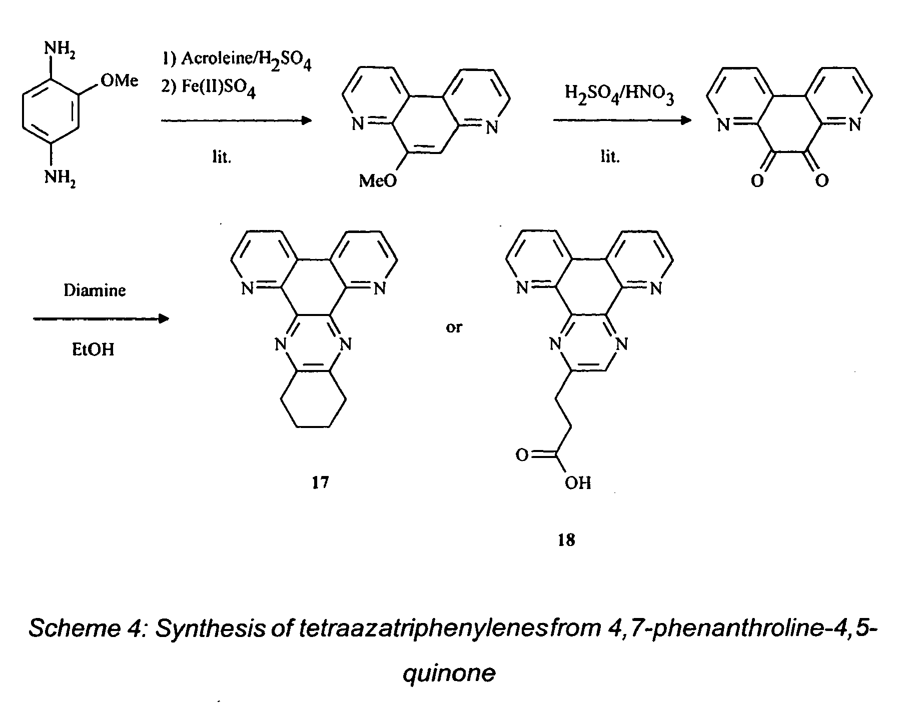

### Example 5

### Synthesis of hexaazatriphenyleneanalogues

Hexamethyl-hexaazatriphenylene(26) and hexaphenyl-hexaazatriphenylene (27) were synthesized according to literature procedures (Kohne, B; Praefcke, K. Liebigs Ann. Chem., 108 (1975), 875). The key step is the condensation of benzil (25) or 2,3-butanedione (24) with hexaaminobenzene (23), which was obtained by reduction of 1,3,5-triamino-2,4,6-trinitrobenzene (22) with phenylhydrazine. 1,3,5-Triamino-2,4,6-trinitrobenzene (22) was synthesized by nitration of 1 ,3,5-trichlorobenzene 19 to give 1,3,5-trichloro-2,4-dinitrobenzene (20). Harsh nitration conditions on dinitro derivative (20) with sulfuric acid/nitric acid gave 1,3,5-trichioro-2,4,6-trinitrobenzene(21) in 76% yield. Subsequent addition of NH₃ gas to (21) gave strongly yellow triamine (22) in 90% yield. Reduction of the trinitro functionalities of 22 with phenylhydrazine gave hexaaminobenzene (23) in 63% yield.

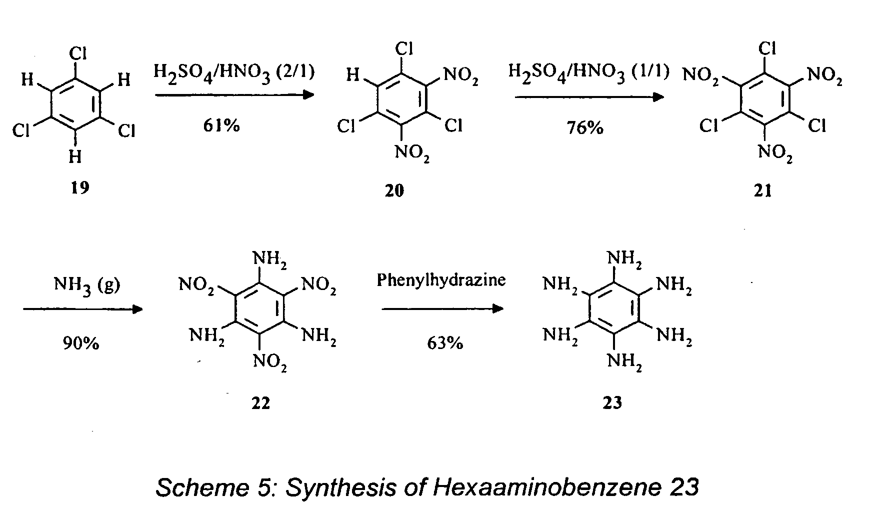

Heaxaaminobenzene 23 reacts with the α-diketones benzil (25) and 2,3-butanedione (24) to give the 2,3,6,7,10,1 1-hexasubstituted dipyrazino[2,3-*f*.2', 3'-h]quinoxalines (26) and (27) in 40-45% yield as depicted in Scheme 6.

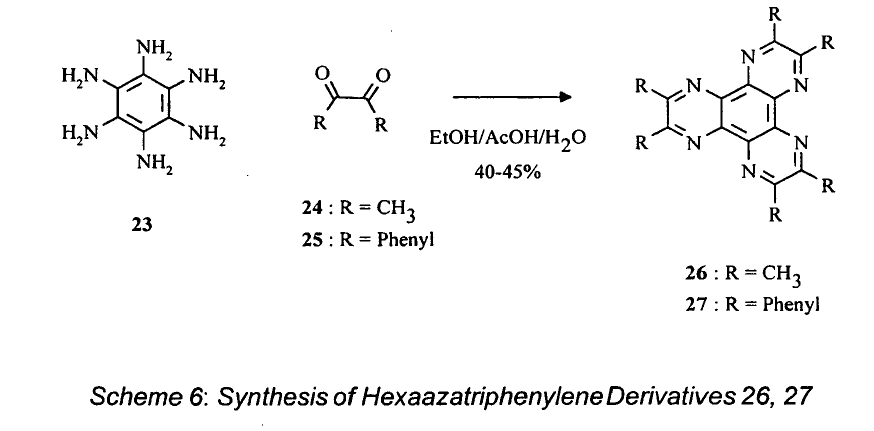

### Example 6

### Synthesis of further red-shifted tetraazatriphenylene from 1,10-phenanthroline-4,5-quinone

### 2,3-(2,3-diaminonaphthalene)-dipyrido[3,2-f:2',3'-h]quinoxaline(28).

Prepared similar to compound (6), starting from 1, 10-phenanthroline-4,5-quinone (5) (0.6 g, 2.9 mmoles) and 2,3-diaminonaphthalene(0.45 g, 2.9 mmoles).

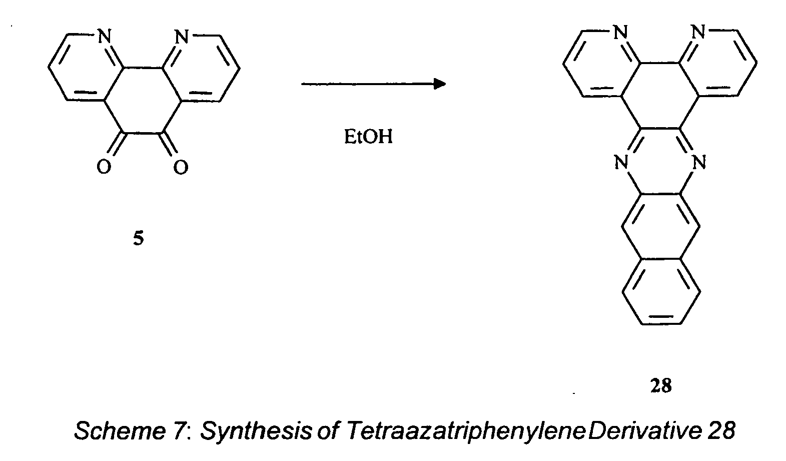

### Example 7

### Synthesis of TBDMS-derivative (32)

A solution of TBDMS-monotrichloroethanolcalix[4]arene (31) (260 mg, 0.081 mmole; prepared according to Scheme 8 as decribed in F.J. Steemers, Sensitizer-Modified Lanthanide Complexes for Time-Resolved Fluoroimmunoassays, Dissertation, University of Twente, The Netherlands (1997), pp. 103-107), tetraazatriphenylene amine (30) (35 mg, 0.093 mmole), 125 µl of 1,5 diazabicyclo[4.3.0]non-5-ene (DBN), and 10 µl of triethylamine in 10 ml of dichloromethane was stirred overnight at room temperature. The solvent was removed *in vacuo* and the reaction mixture was purified by preparative silica plate with 10% methanol/dichloromethane as eluent. Yield of calix[4]arene (32) was 14%.

A solution of potassium carbonate (16 mg, 0.12 mmole) in 3 ml of water was added to a refluxing solution of calix[4]arene (32) (40 mg, 0.012 mmole) in methanol (10 ml). After 1 h of refluxing, the mixture was poured into water (50 ml), after which the pH was adjusted to about 2 with 1 N hydrochloric acid. The water layer was extracted with dichloromethane to give, after removal of the solvent *in vacuo,* the triacid as a white solid. Yield 50%. A solution of 0.5 g of this triacid calix[4]arene in trifluoroacetic acid (TFA/H₂O; 9/1, 25 ml) was stirred overnight after which toluene (50 ml) was added. The solution was concentrated *in vacuo,* after which fresh toluene (50 ml) was added and the mixture was concentrated *in vacuo.* This procedure was repeated three times. The crude white solid was dissolved in a minimum amount of water and acetone was added dropwise to this solution till the product started to precipitate. After the suspension was stirred for 30 min, the product was filtered, freeze-dried, and analyzed. Prior to the luminescence measurements, the solution of this compound in water was dialyzed to remove excess of trifluoroacetic acid and was freeze-dried again.

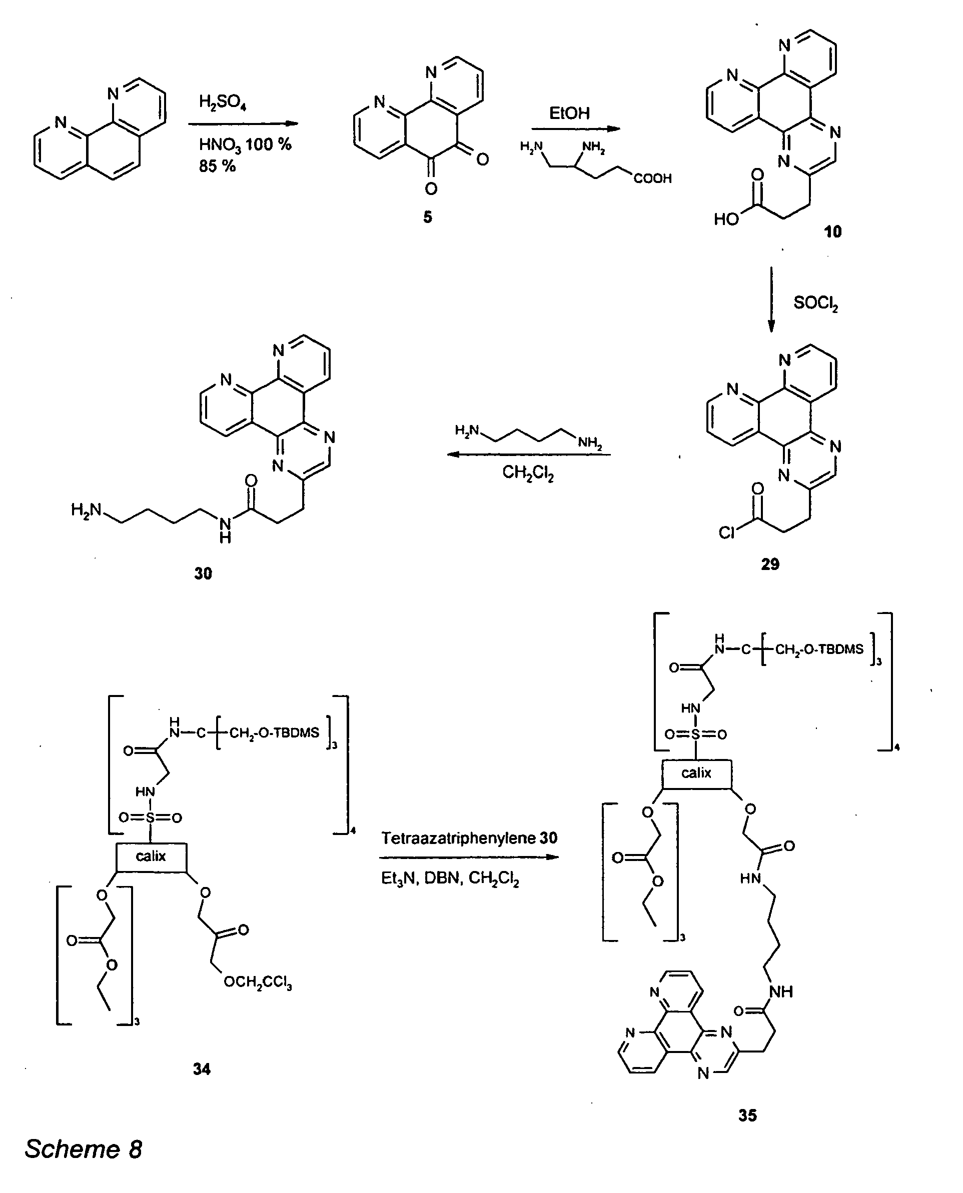

### Example 8

### Preparation of Rare-Earth Complexes

Rare-earth complexes have been prepared for all compounds described above. The complexes have been obtained by adding a 1:2 (mole lanthanide/mole ligand) amount of lanthanide salt (generally the nitrate was used) to a solution of ligand in acetonitrile, which had been kept over molecular sieves prior to use. Titration experiments demonstrated that a 2:1 complex is formed between the ligand and the lanthanide ion. The luminescence of the sensitized lanthanide is at a maximum for the 2:1 composition of the complex. The following complexes have been prepared: Tb³⁺, Eu³⁺, Dy³⁺, Sm³⁺, Yb³⁺, Nd³⁺, and Er³⁺.

### Example 9

### Photophysical measurements

For the complexes prepared as described in Example 8 absorption spectra and luminescence emission and excitation spectra have been recorded. In addition luminescence lifetime measurements have been done, in aerated and deaerated solution. Quantum yields were determined for the Tb- and Eu-complexes, using quinine sulfate in 1 M sulfuric acid (with a reported quantum yield of 0.546) as reference.

The effective sensitization of the lanthanide ions is clear from the luminescence excitation spectra: when the rare earth luminescence is monitored, variation of the excitation wavelength shows a spectrum which is similar to the absorption spectrum of the organic ligand. In most cases the absorption spectrum of the ligand is even enhanced and somewhat red-shifted upon complexation with the rare earth ion, which is favorable for most applications. Sensitized excitation results in a tremendous improvement of the luminescence intensity, due to the typically 1,000-10,000 fold higher absorption cross sections of the ligand, as compared to the lanthanide ion. The energy transfer rate is high, since deaeration does not have any great impact on most measured luminescence intensities and lifetimes.

Quantitative data for a number of complexes are summarized in Tables 1-3.

**Table 1. Photophysical data of (aza)triphenylenes and their Eu³⁺ and Tb³⁺ complexes in acetonitrile.**

| **compound** | **¹E₀₀** (cm⁻¹) (nm) | ε (M⁻¹·cm⁻¹) | **³E₀₀** (cm⁻¹) | **Φ_{Eu}** (λₑₓ) | **Φ_{Tb}** (λₑₓ) |
|---|---|---|---|---|---|
| **triphenylene** | 29900 (334) | 355 | 23300 | < 0.001 | < 0.001 |
| **3** | 29700 (337) | 1290 | 23800 | 0.41 (337) | 0.55 (337) |
| **8** | 28800 (347) | 6440 | 23400 | 0.37 (343) | 0.62 (343) |
| **9** | 26500 (377) | 9160 | ~18500 | 0 | 0 |
| **6** | 29400 (340) | 3800 | 23800 | 0.41 (335) | 0.67 (335) |
| **7** | 27300 (366) | 5520 | 21700 | 0.25 (355) | 0.14 (355) |
| **26** | 29800 (336) ^{b} | 9300 | 22300 | 0.001 (345) | <0.001 (345) |
| **27** | 27200 (367)^{b,c} | >9000 ^{c} | ~19100 | 0 | 0 |
| **16** | 28600 (350) | 6630 | 22500 | 0.29 (340) | 0.30 (340) |
| **11** | 29400 (340) | 8500 | 23600 | 0.43 (337) | 0.51 (337) |

| | | | | | |
|---|---|---|---|---|---|
| ^{a} Not determined. ^{b} Shoulder on broad absorption. ^{c} Saturated solution. ^{d} Abbreviations: ¹E₀₀ = energy of the lowest electronic singlet 0-0 transition (determined from the absorption spectrum); ε = extinction coefficient of the electronic singlet 0-0 transition; ³E₀₀ = energy of the triplet 0-0 transition (determined from the 77 K phosphorescence spectrum); Φ = luminescence quantum yield, determined with reference to the quinine sulfate standard. | | | | | |

**Table 2. Luminescence lifetimes of azatriphenylene-lanthanide complexes in acetonitrile; λₑₓ= 308 nm.**

| **compound** | τ **^{Eu,ox}** (ms) | τ **^{Eu,deox}** (ms) | τ **^{Tb,ox}** (ms) | τ **^{Tb,deox}** (ms) |
|---|---|---|---|---|
| **3** | 1.20 | 1.19 | 1.31 | 1.32 |
| **8** | 1.03 | 1.02 | 1.20 | 1.21 |
| **6** | 1.04 | 0.98 | 1.43 | 1.36 |

| | | | | |
|---|---|---|---|---|
| ^{a} Abbreviations: τₒₓ is luminescence lifetime in oxygen containing solution, τ^{deox} is luminescence lifetime in deoxygenated solution. | | | | |

Compounds 9, 26, 27 and 28 have insufficient triplet energy to sensitize the Eu³⁺- and Tb³⁺-ions, which emit in the visible part of the spectrum and therefore require sensitization in the UV- or blue part of the spectrum. The "red-shifted" sensitizers, however, do give energy transfer to near-IR emitting rare earth ions like Yb³⁺, Nd³⁺, and Er³⁺.

**Table 3. Photophysical data of (aza)triphenylene based Yb³⁺, Nd³⁺, and Er³⁺ complexes in acetonitrile.**

| **compound** | **¹E₀₀**(cm⁻¹) (nm) | **τ^{Yb,ox} (µs)** | **τ^{Nd,ox} (µs)** | **τ^{Er,ox} (µs)** |
|---|---|---|---|---|
| **3** | 29700(337) | 7.4 | 0.38 | 1.7 |
| **9** | 26500(377) | 6.2 | 0.33 | 2.0 |
| **6** | 29400(340) | 7.5 | 0.29 | 2.3 |
| **28** | 21500(465) | 7.9 | 0.32 | 2.5 |

| | | | | |
|---|---|---|---|---|
| ^{a} Abbreviations: ¹E₀₀ = energy of the lowest electronic singlet 0-0 transition (determined from the absorption spectrum); τ ^{ox} (µs) = lifetime of the rare earth ion luminescence (longest, generally main, component) in oxygen-containing solution. | | | | |

## Claims

1. A complex comprising a rare-earth metal ion and a complexing moiety, the complexing moiety being of the general formula 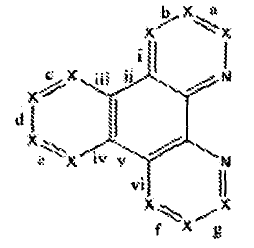 wherein
- X is independently CH or N,
- the bonds a, b, c, d, e, f and g, and the combination of bonds i/ii/iii and iv/v/vi are optionally condensed with a benzene group or a condensed aromatic moiety wherein aromatic carbon atoms may be replaced by nitrogen atoms, and
- the complexing moiety may be substituted with C₁₋₆ alkyl, C₂₋₆ alkenyl, C₂₋₆ alkynyl, C₃₋₄ alkylene, CN, halogen, C₁₋₃ alkyl-COOH, NO₂, NH₂ or a pending group for further functionalisation or complexation, the pending group comprising a coupling moiety being selected from the group consisting of isothiocyanate, activated ester, epoxide, acetic anhydride, maleimide, halogenoacetamide, amino, thiol, hydrazide, aldehyde, isocyanate, acid chloride, hydroxy, a group for forming a complex with a rare-earth metal ion where the group is a nitrogen-containin, a sulfur-containing or a phosphorous-containing group or calixarene.

2. The complex of claim 1, wherein at least one of the groups X is N.

3. The complex of claim 1, wherein the complexing moiety is of the general formula 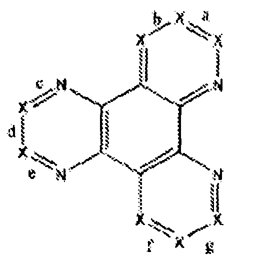 wherein
- X is independently CH or N,
- the bonds a, b, c, d, e, f, and g are optionally condensed with a benzene group or a condensed aromatic moiety wherein aromatic carbon atoms may be replaced by nitrogen atoms, and
- the complexing moiety may be substituted with C₁₋₆ alkyl, C₂₋₆ alkenyl, C₂₋₆ alkynyl, C₃₋₄ alkylene, CN, halogen, C₁₋₃ alkyl-COOH, NO₂, NH₂ or a pending group for further functionalisation or complexation, the pending group comprising a coupling moiety being selected from the group consisting of isothiocyanate, activated ester, epoxide, acetic anhydride, maleimide, halogenoacetamide, amino, thiol, hydrazide, aldehyde, isocyanate, acid chloride, hydroxy, a group for forming a complex with a rare-earth metal ion where the group is a nitrogen-containin, a sulfur-containing or a phosphorous-containing group or calixarene.

4. A process for preparing the complex of any one of claims 1 to 3 comprising the step of bringing into contact a solution of a rare-earth salt and a solution of the complexing moiety of claim 1.

5. An optical device or a diagnostic kit comprising the complex of any one of claims 1 to 3.

## Patentansprüche

1. Komplexe mit einem Seltenerdmetall-Ion und einer komplexbildenden Komponente, wobei die komplexbildende Komponente durch die allgemeine Formel 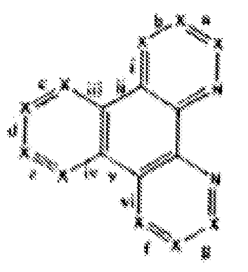 dargestellt ist, wobei
- X unabhängig CH oder N ist,
- die Bindungen a, b, c, d, e, f und g sowie die Kombination aus Bindungen i/ii/iii und iv/v/vi mit einer Benzolgruppe oder einer kondensierten, aromatischen Komponente optional kondensiert werden, wobei aromatische Kohlenstoffatome durch Stickstoffatome ersetzt werden können, und
- wobei die komplexbildende Komponente gegen C₁₋₆ Alkyl, C₂₋₆ Alkenyl, C₂₋₆ Alkynyl, C₃₋₄ Alkylen, CN, Halogen, C₁₋₃ Alkyl-COOH, NO₂, NH₂ oder eine "Pending Group" (Seitenkette) zur weiteren Funktionalisierung oder Komplexierung ausgetauscht werden kann, wobei die "Pending Group" eine Kopplungskomponente aufweist, welche aus der aus Isothiocyanat, aktiviertem Ester, Epoxid, Essigsäureanhydrid, Maleimid, Halogenoacetamid, Amino, Thio, Hydrazid, Aldehyd, Isocyanat, Säurechlorid, Hydroxy bestehenden Gruppe gewählt wird, einer Gruppe zur Bildung eines Komplexes mit einem Seltenerdmetall-Ion, wobei die Gruppe eine stickstoffhaltige, schwefelhaltige oder eine phosphorhaltige Gruppe oder Calixaren ist.

2. Komplex nach Anspruch 1, wobei mindestens eine der Gruppen X N ist.

3. Komplex nach Anspruch 1, wobei die komplexbildende Komponente die allgemeine Formel 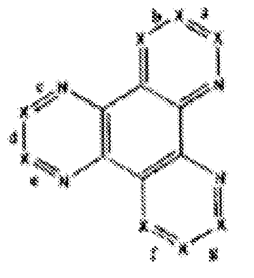 aufweist, wobei
- X unabhängig CH oder N ist,
- die Bindungen a, b, c, d, e, f und g mit einer Benzolgruppe oder einer kondensierten, aromatischen Komponente optional kondensiert werden, wobei aromatische Kohlenstoffatome durch Stickstoffatome ersetzt werden können, und
- wobei die komplexbildende Komponente gegen C₁₋₆ Alkyl, C₂₋₆ Alkenyl, C₂₋₆ Alkynyl, C₃₋₄ Alkylen, CN, Halogen, C₁₋₃ Alkyl-COOH, NO₂, NH₂ oder eine "Pending Group" (Seitenkette) zur weiteren Funktionalisierung oder Komplexierung ausgetauscht werden kann, wobei die "Pending Group" eine Kopplungskomponente aufweist, welche aus der aus Isothiocyanat, aktiviertem Ester, Epoxid, Essigsäureanhydrid, Maleimid, Halogenoacetamid, Amino, Thio, Hydrazid, Aldehyd, Isocyanat, Säurechlorid, Hydroxy bestehenden Gruppe gewählt wird, einer Gruppe zur Bildung eines Komplexes mit einem Seltenerdmetall-Ion, wobei die Gruppe eine stickstoffhaltige, schwefelhaltige oder eine phosphorhaltige Gruppe oder Calixaren ist.

4. Verfahren zur Herstellung des Komplexes nach einem der Ansprüche 1 bis 3, wonach eine Lösung aus einem Salz der seltenen Erden und eine Lösung aus der komplexbildenden Komponente von Anspruch 1 in Kontakt gebracht werden.

5. Optische Vorrichtung oder Diagnosekit mit dem Komplex nach einem der Ansprüche 1 bis 3.

## Revendications

1. Complexe comprenant un ion métallique du groupe des terres rares et un radical complexant, le radical complexant répondant à la formule générale : 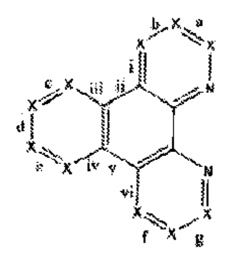 dans laquelle :
- X représente indépendamment CH ou N,
- les liaisons a, b, c, d, e, f et g, et la combinaison des liaisons i/ii/iii et iv/v/vi sont éventuellement condensées avec un groupement benzène ou un radical aromatique condensé, dans lequel des atomes de carbone aromatiques peuvent être remplacés par des atomes d'azote, et
- le radical complexant peut être substitué par un alkyle en C₁₋₆, un alcényle en C₂₋₆, un alcynyle en C₂₋₆, un alkylène en C₃₋₄, CN, un halogène, un alkyle(C₁₋₃)-COOH, NO₂, NH₂ ou un groupement pendant en vue d'une fonctionnalisation ou d'une complexation ultérieure, le groupement pendant comprenant un radical de couplage qui est choisi dans le groupe constitué des radicaux isothiocyanate, ester actif, époxyde, anhydride acétique, maléimide, halogénoacétamide, amino, thiol, hydrazide, aldéhyde, isocyanate, chlorure d'acide, hydroxy, un groupement destiné à la formation d'un complexe avec un ion métallique du groupe des terres rares, le groupement en question étant un groupement contenant de l'azote, un groupement contenant du soufre ou un groupement contenant du phosphore, ou un calixarène.

2. Complexe selon la revendication 1, dans lequel au moins l'un des groupements X représente N.

3. Complexe selon la revendication 1, dans lequel le radical complexant répond à la formule générale : 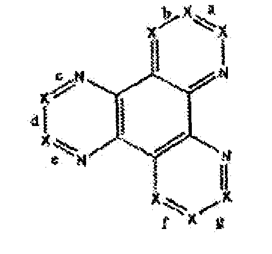 dans laquelle :
- X représente indépendamment CH ou N,
- les liaisons a, b, c, d, e, f et g sont éventuellement condensés avec un groupement benzène ou un radical aromatique condensé, dans lequel les atomes de carbone aromatiques peuvent être remplacés par des atomes d'azote, et
- le radical complexant peut être substitué par un alkyle en C₁₋₆, un alcényle en C2-6, un alcynyle en C₂₋₆, un alkylène en C₃₋₄, CN, un halogène, un alkyle(C₁₋₃)-COOH, NO₂, NH₂ ou un groupement pendant en vue d'une fonctionnalisation ou d'une complexation ultérieure, le groupement pendant comprenant un radical de couplage qui est choisi dans le groupe constitué des radicaux isothiocyanate, ester actif, époxyde, anhydride acétique, maléimide, halogénoacétamide, amino, thiol, hydrazide, aldéhyde, isocyanate, chlorure d'acide, hydroxy, un groupement destiné à former un complexe avec un ion métallique du groupe des terres rares, le groupement en question étant un groupement contenant de l'azote, un groupement contenant du soufre ou un groupement contenant du phosphore, ou un calixarène.

4. Procédé pour préparer le complexe selon l'une quelconque des revendications 1 à 3, comprenant l'étape consistant à mettre en contact une solution d'un sel d'élément du groupe des terres rares et une solution du radical complexant selon la revendication 1.

5. Dispositif optique ou trousse de diagnostic, comprenant le complexe selon l'une quelconque des revendications 1 à 3.
